(19) Europäisches Patentamt
European Patent Office
Office européen des brevets

(11) Publication number: **0 336 368 B1**

# (12) EUROPEAN PATENT SPECIFICATION

(45) Date of publication of patent specification: **01.02.95**

(51) Int. Cl.6: **C07C 233/00**, C07C 255/00, C07K 5/00, A61K 38/55, A61K 31/00, C07D 263/44, C07K 5/06, C07C 237/20, C07C 255/42

(21) Application number: **89105882.8**

(22) Date of filing: **04.04.89**

## (54) N2-(1-carboxy-3-phenylpropyl)-L-lysine derivative and process of producing lysinopril using the compound.

(30) Priority: **04.04.88 JP 83632/88**

(43) Date of publication of application:
**11.10.89 Bulletin 89/41**

(45) Publication of the grant of the patent:
**01.02.95 Bulletin 95/05**

(84) Designated Contracting States:
**BE CH DE ES FR GB IT LI**

(56) References cited:
**EP-A- 0 160 307**
**EP-A- 0 168 769**
**US-A- 4 652 668**

(73) Proprietor: **KANEGAFUCHI KAGAKU KOGYO KABUSHIKI KAISHA**
**2-4 Nakanoshima 3-chome**
**Kita-ku**
**Osaka-shi**
**Osaka-fu 530 (JP)**

(72) Inventor: **Inoue, Kenji**
**1-13-36-301, Kita Honjyo**
**Harima-cho**
**Kako-gun**
**Hyogo (JP)**
Inventor: **Matsumoto, Mitsunori**
**570, Kamijyosui**
**Jyosui-ken**
**Nakatsu-shi**
**Oita (JP)**
Inventor: **Takahashi, Satomi**
**13-13, Shinwadai 1-chome**
**Tarumi-ku**
**Kobe-shi**
**Hyogo (JP)**

(74) Representative: **VOSSIUS & PARTNER**
**Postfach 86 07 67**
**D-81634 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

This invention relates to an $N^2$-(1-cyano-3-phenylpropyl)-L-lysine derivative (V), a process of effectively producing $N^2$-(1-(S)-carboxy-3-phenylpropyl)-L-lysyl-L-proline (lysinopril) (VIII) using the aforesaid derivative, and an intermediate thereof.

Lysinopril (VIII) is a compound expected to be utilized as an antihypotensive agent owing to its excellent angiotensine conversion enzyme (ACE) inhibiting activity.

BACKGROUND OF THE INVENTION

As a process of producing lysinopril (VIII), there is known a process of reacting $\beta$-phenylpropionaldehyde and $N^6$-tert-butoxycarbonyl-L-lysine (IX) with potassium cyanide (Strecker reaction) to form a compound shown by formula (X) below, successively treating the compound (X) with hydrogen chloride/methanol and an acid ion exchange resin to form a diester derivative shown by formula (XI) shown below, and further alkali-hydrolyzing the diester derivative (XI) as shown in the following reaction formula (JP-A-58-113158) (the term "JP-A" as used herein means an "unexamined published Japanese patent application");

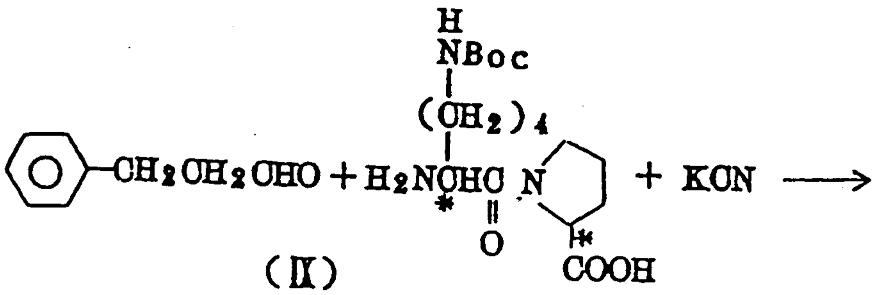

(IX)

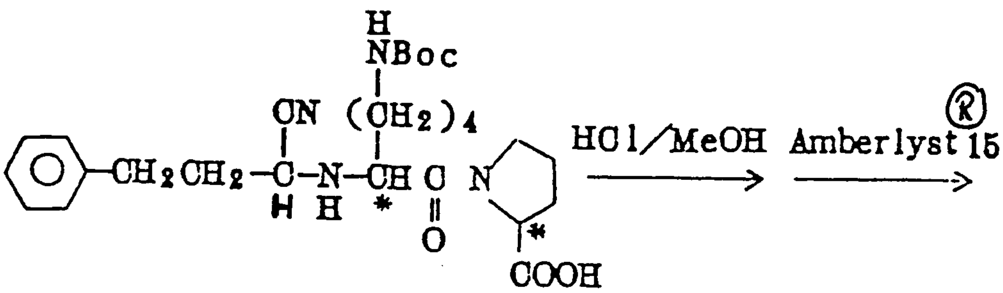


(X)

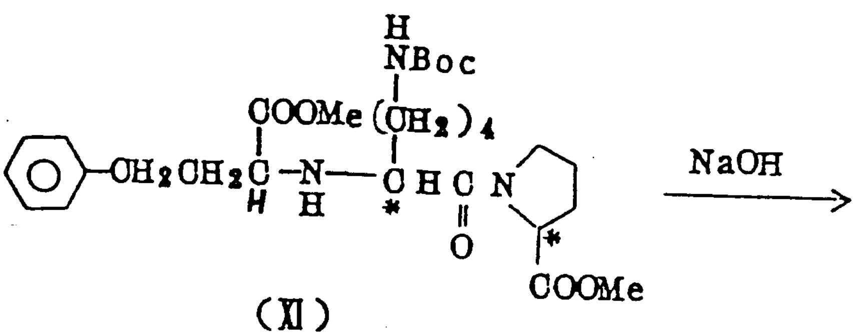


(VIII)

(XI)

(BOC = t-butoxycarbonyl)

Also known is a process of reacting the N-carboxy anhydride of $N^6$-trifluoroacetyl-L-lysine and L-proline to provide $N^6$-trifluoroacetyl-L-lysyl-L-prolineshown by formula (XII) below, reductively alkylating the proline derivative (XII) with ethyl $\alpha$-oxo-$\gamma$-phenylbutyrate (XIII) to form $N^6$-(1-ethoxycarbonyl-3-phenylpropyl)-L-lysyl-L-proline shown by formula (XIV) below, and then alkali-hydrolyzing the proline derivative (XIV) as shown by the following reaction formula (JP-A-61-36297).

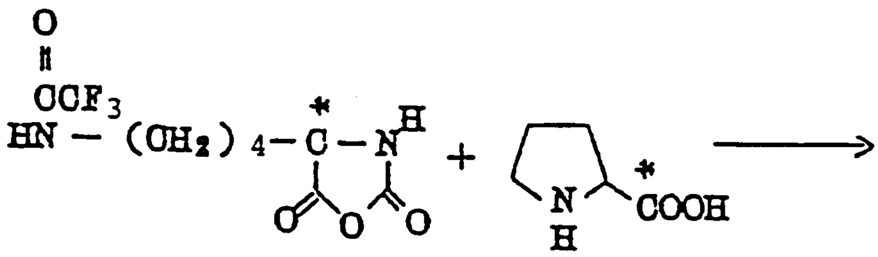

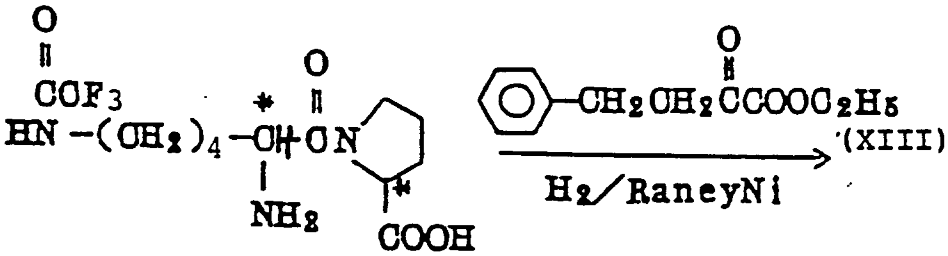


(XII)

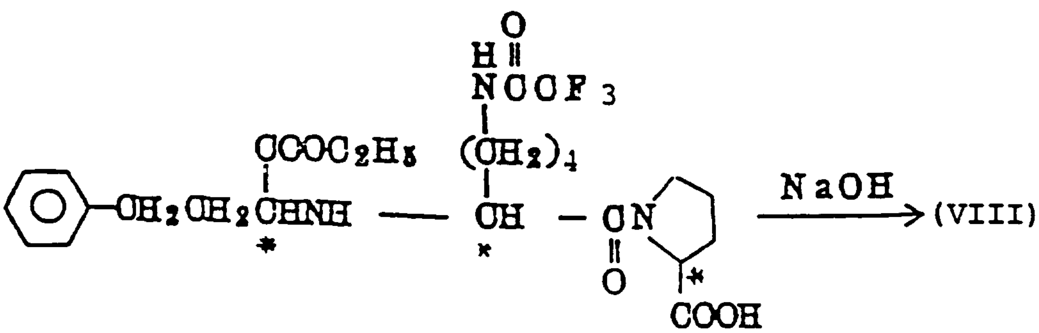


(XIV)

US-A-4,652,668 discloses a process involving:

a) reacting $\beta$-phenylpropionaldehyde with an amino acid in the presence of a cyanating agent

b) separating the resulting diastereomers

c) condensing proline to the separated diastereomers of Step a)

d) converting the compound of Step c to pharmaceuticals.

However, the materials or pigments used in the aforesaid processes except $\beta$-phenylpropionaldehyde, L-proline, and potassium cyanide have the following disadvantages: That is, they have complicated structures, they are not always easily available, they are expensive and require a considerable amount of steps for the synthesis thereof. Also, for obtaining an isomer having the absolute configuration (S,S,S) which is required in order to obtain lysinopril which exhibits a satisfactory angiotensine conversion enzyme inhibitor activity (ACEI activity), it is desirable to separate the starting materials, including the intermediate, in order to obtain the final product in high configurative purity since the starting materials are expensive and may be easily separated through an optical resolution. Thus, with the aforesaid processes there are economical difficulties, also with respect to their operability within a production process of lysinopril in a commercially practicable scale.

The inventors previously discovered a process of efficiently producing $\alpha$-(1-carboxyethyl)amino-$\gamma$-phenylbutyric acid ethyl esters which are very useful as intermediates for producing various compounds exhibiting ACEI activity by using the Strecker reaction and employing inexpensive starting materials as described in Japanese Patent Application No. 62-204860.

SUMMARY OF THE INVENTION

As the result of investigations for developing a process of efficiently producing lysinopril (VIII) based on the aforesaid technique, the inventors have discovered a novel process for efficiently producing lysinopril (VIII) using, as an intermediate, an $N^2$-(1-cyano-3-phenylpropyl)-L-lysine derivative (V) which can be easily produced by subjecting $\beta$-phenylpropionaldahyde and an L-lysine derivative (IV), which are inexpensive and easily available raw materials, to a so-called Strecker reaction in the presence of a cyanating agent and thus have succeeded in accomplishing the present invention.

The reaction formula of the process of this invention is as follows.

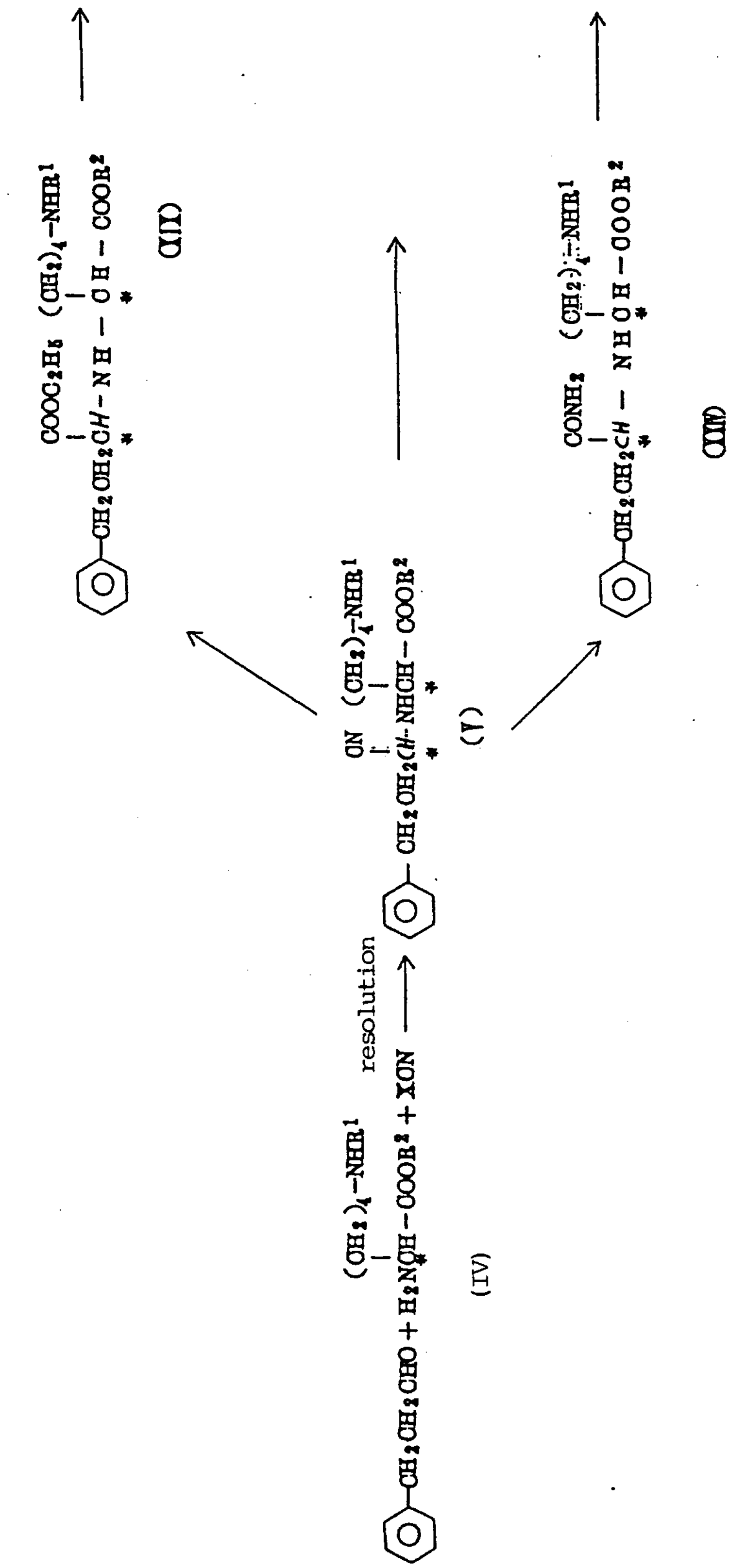

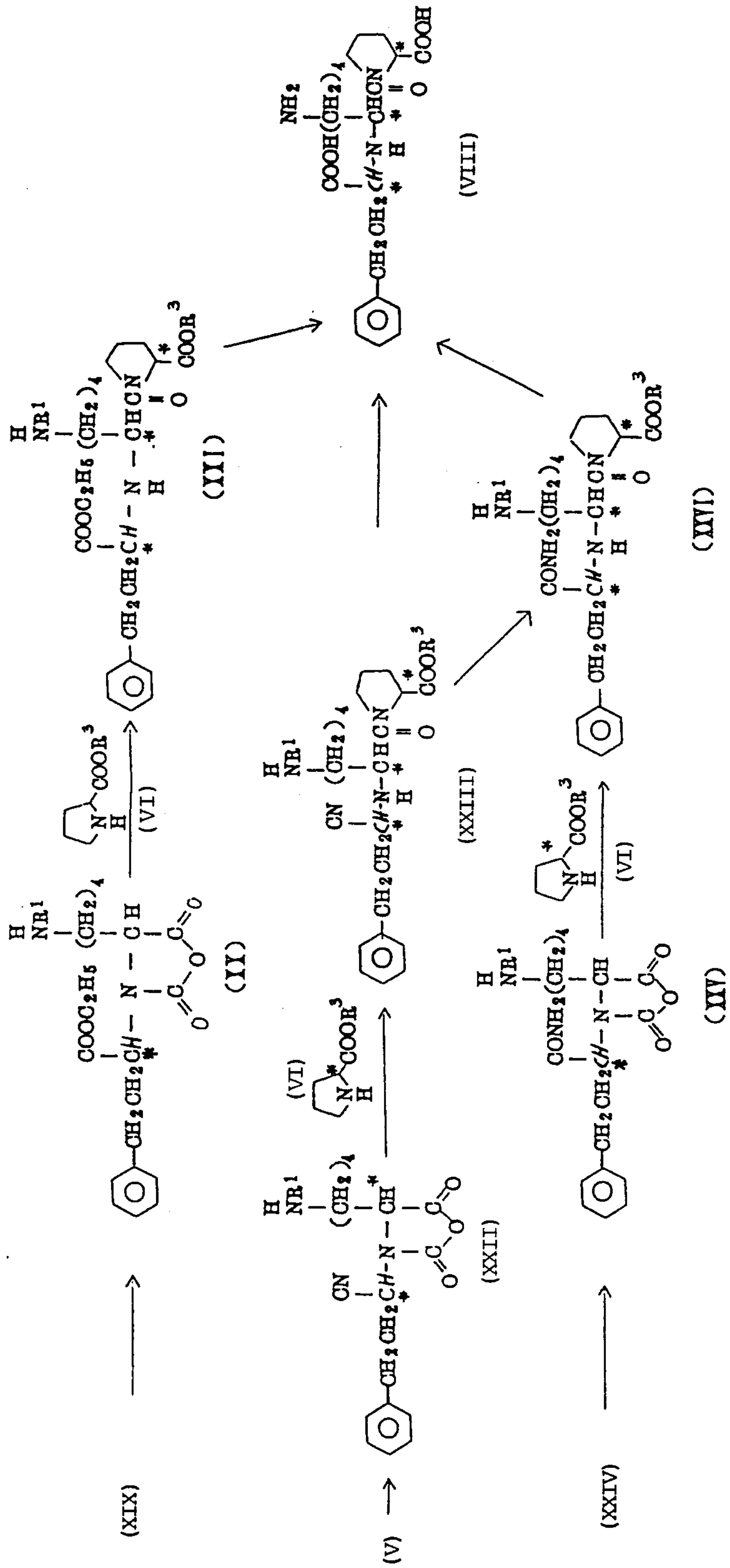


In the aforesaid reaction formula, $R^1$ represents an acyl type or urethane type protective group; $R^2$ represents a hydrogen atom, an alkyl group, or an aralkyl group; and the mark * represents the S-position of the asymmetric carbon atom. X in XCN represents the moiety of a cyanating agent attached to the CN group.

That is, it has now been clarified that an $N^2$-(1-substituted-3-phenylpropyl)-L-lysyl-L-proline (XXIII) or (XXVI) can be produced by treating an $N^2$-(1-cyano-3-phenylpropyl)-L-lysine derivative (V), (which is a novel

compound capable of being easily produced from phenylpropionaldehyde, a L-lysine derivative, and a cyanating agent XCN) as it is or after converting (V) to an amide derivative (XXIV) by an acid hydrolysis with phosgene, etc., to form an N-carboxy anhydride (XXII) or (XXV), and reacting the N-carboxy anhydride and a L-proline (VI) in the presence of a base; that the compound (XXIII) or (XXVI) can be easily converted into lysinopril (VIII); that an $N^2$-(1-ethoxy - carbonyl-3-phenylpropyl)-L-lysine derivative (XIX) can be produced by treating an $N^2$-(1-cyano-3-phenylpropyl)-L-lysine derivative (V) with HCl/methanol and hydrolyzing the imidate thus formed; and that an $N^2$-(1-ethoxycarbonyl-3-phenylpropyl)-L-lysyl-L-proline derivative (XXI) can be easily obtained by treating the $N^2$-(1-ethoxycarbonyl-3-phenylpropyl)-L-lysine derivative (XIX) with phosgene, etc., to provide an N-carboxy anhydride (XX), and then reacting the N-carboxy anhydride (XX) and a proline (VI) under basic conditions.

Also, it has been clarified that lysinopril (VIII) can be advantageously produced by that the desired (1S) compound can be predominantly produced in asymmetric induction by controlling the synthesis condition of the $N^2$-(1-cyano-3-phenylpropyl)-L-lysine derivative (V) in the aforesaid case as compared with the (1R) compound, and that the (1S) L-lysine derivative (IV) can be easily separated from the (1R) L-lysine derivative (IV), and the invention has been accomplished based on the finding.

## DETAILED DESCRIPTION OF THE INVENTION

The invention will now be explained in detail.

Examples for the alkyl groups, alone or mentioned in conjunction with other substituents as mentioned in this invention are lower alkyl groups with 1 to 10, preferably 1 to 5 carbon atoms, straight chain or branched. The term aryl, alone or in conjunction with other substituents (e.g. aralkyl), is preferably meant to describe cyclic aromates with 5 to 7, more preferably with 6 carbon atoms (e.g. the phenyl group). As the lysine component (IV) in the production of the $N^2$-(1-cyano-3-phenylpropyl)-L-lysine derivate (V), L-lysine derivatives in which the $\epsilon$-amino group is protected by protective groups which are usually employed in peptide synthesis reactions, and the esters and salts can be used. Examples for the protective group which may be used for the purpose are substituted oxycarbonyl groups such as a tertiary butyloxycarbonyl group, a benzyloxycarbonyl group, a p-nitrobenzyloxycarbonyl group, etc.; an urethane type protective group such as an isobornyloxycarbonyl group, etc.; and an acyl type protective group such as a trifluoroacetyl group, a formyl group, a phthaloyl group, etc.

Examples for the salts of the protected (S)-lysine derivative with a base are alkali metal salts such as lithium salts, sodium salts, potassium salts, etc., and quaternary ammonium salts thereof. Examples for the esters of the protected (S)-lysine derivative are esters which are usually utilized for the synthesis of peptides, such as the alkyl esters (e.g., methyl, ethyl, n-propyl, iso-propyl, n-butyl, tert-butyl, cyclohexyl, and trichloroethyl) and the aryl esters (e.g., phenyl).

Specific examples for the cyanating agent XCN are compounds which are usually used for the synthesis of aminonitrile, such as hydrocyanic acid, sodium cyanide, potassium cyanide, acetone cyanhydrin, trimethylsilyl cyanide, etc.

When reacting the lysine derivative (IV) and the cyano compound XCN, it is a matter of routine to choose the most advantageous combination. For example, in the case of using the salt of a protected (S)-lysine derivative or of an ester thereof with an acid, a combination with an alkali metal cyanide is most effective, and in the case of using the salt of a protected L-lysine with a base or the ester of the protected L-lysine with a base, a combination thereof and hydrocyanic acid, acetone cyanhydrin, or trimethylsilyl cyanide is most effective. However, the invention is not limited to such combinations in which the acid-base balance is being controlled by adding an acid or a base to the reaction system. Various other combinations may be also employed.

The $N^2$-(1-cyano-3-phenylpropyl)-L-lysine derivative (V) may be prepared for example, by

i) a process of simultaneously reacting the three components, i.e., phenylpropionaldehyde, an L-lysine component (IV), and a cyano compound XCN;

ii) a process of first synthesizing a cyanhydrin compound from phenylpropionaldehyde and a cyano compound in a known manner and then reacting the cyanhydrin with a lysine component (IV); and

iii) a process of first obtaining a so-called Schiff base from phenylpropionaldehyde with a lysine component (IV), and then adding thereto a cyano compound XCN.

Also the aforesaid processes can be carried out under usual conditions which are generally employed in an ordinary synthesis of an aminonitrile.

That is, in process i),the reaction is carried out at a temperature range of from 0 °C to 50 °C. In process ii), cyanohydrin is obtained by reacting phenylpropionaldehyde and hydrocyanic acid in the presence of a base at a temperature of from 0 °C to 60 °C or reacting a phenylpropionaldehyde-sodium hydrogensulfite

addition product and an alkali metal cyanide at about room temperature and reacting the cyanohydrin and the lysine component at a temperature of from 0°C to 50°C. In process iii), the Schiff base is formed by reacting phenylpropionaldehyde and the lysine component in an anhydrous solvent in the presence of an ordinary dehydrating agent such as molecular sieve and anhydrous magnesium sulfate at a temperature of from 0°C to 100°C, and preferably from 10°C to 30°C, and then the Schiff base is reacted with hydrocyanic acid, a cyanide, trimethylsilyl cyanide, etc., under cooling to provide the aminonitrile compound (V).

The aforesaid reactions, except for the case of forming the Schiff base in process iii), can be usually carried out in water, an organic solvent, or a mixture thereof.

Examples for the organic solvent are alcohols such as methanol, ethanol, isopropanol, etc.; nitriles such as acetonitrile, propionitrile, etc.; ethers such as diethyl ether, dioxane, tetrahydrofuran, etc.; amides such as dimethylformamide, hexamethylphosphoramide, etc.; and halogenated hydrocarbons such as methylene chloride, chloroform, etc.

The conditions for predominantly obtaining the (S,S) isomers (as opposed to the (R,S) isomers) in the reaction differ according to the process being employed from the aforesaid processes i), ii), and iii), but in the case of using, for example, process i), the $N^2$-(1(S)-cyano-3-phenylpropyl)-L-lysine derivative can be predominantly obtained over the (1R) isomer by successively mixing phenylpropionaldehyde, sodium cyanide, and the L-lysine component in a protonic solvent such as water, methanol, ethanol, isopropanol, etc., solely or as a mixture thereof with other solvents and by stirring at a temperature of from -40°C to 80°C, and preferably from 0°C to 50°C, for from 50 hours to 5 minutes.

For obtaining the pure (1S) isomer out from the mixture of the isomers of $N^2$-(1-cyano-3-phenylpropyl)-L-lysine thus obtained, various methods which are commonly employed for a separation (resolution) of a diasteromer can be employed and, for example, the crystals of the (1S) isomer can be easily obtained by recrystallizing from a mixture of water and methanol.

The conversion of the $N^2$-(1(S)-cyano-3-phenylpropyl)-L-lysine derivative (V) into the amide derivative (XXIV) can be easily carried out by using a mineral acid such as hydrochloric acid, sulfuric acid, etc., according to a per se known manner. The conversion can be attained by carrying out the reaction at a temperature of from -10°C to 80°C, and preferably from 0°C to 50°C, for from 40 hours to 5 minutes. In this case, for inhibiting the occurence of side-reaction such as cleavage of the peptide bond, etc., it is preferred to carry out the reaction at a low temperature of from 0°C to 30°C, using sulfuric acid as the mineral acid in the presence of water in an amount of from 0.1% to 100%, and preferably from 1% to 50% of the sulfuric acid.

The conversion of the $N^2$-(1(S)-cyano-3-phenylpropyl)-L-lysine derivative (V) into the ethyl ester derivative (XIX) can be carried out by an ordinary process of converting a cyano group into the ester group thereof. For example, the $N^2$-(1(S)-ethoxycarbonyl-3-phenylpropyl)-L-lysine derivative (XIX)can be obtained by reacting the L-lysine derivative (V) in a saturated methanol solution of dry hydrogen chloride gas at a temperature of from -30°C to 30°C, and preferably from 0°C to 10°C, for from 40 hours to 1 hour, and hydrolyzing the imidate thus formed with water.

The conversion of the $N^2$-(1(S)-substituted-3-phenylpropyl)-L-lysine derivative (XIX), (V), or (XXIV) to the corresponding N-carboxy anhydride can be carried out by the same procedure as in the case of a usual synthesis of the N-carboxy anhydride of an $\alpha$-amino acid, e.g. as described in JP-A-57-175152 and 62-48696, after decomposing the ester by an ordinary method using an acid, a base, or by hydrogenolysis when the L-lysine component has no ester group.

That is, the aforesaid conversion can be easily performed by refluxing the L-lysine derivative (XIX), (V), or (XXIV) in methylene chloride containing phosgene, or by heating the derivative with trichloromethyl chloroformate in an inert solvent in the presence of a small amount of active carbon.

The peptide bond forming reaction of the N-carboxy anhydride thus obtained with the L-proline (VI) can be easily achieved by mixing the L-proline (VI) and the N-carboxy anhydride in the presence of a base as described in JP-A-62-48696.

As the base for use in the aforesaid reactions, one may employ inorganic bases such as hydroxides, carbonates, and hydrogencarbonates of alkali metals such as lithium, sodium, and potassium; hydroxides of alkaline earth metals such as calcium and magnesium; as well as amines excluding primary amines, e.g., secondary amines such as dimethylamine, diethylamine, diethanolamine, dichlorohexylamine, etc., tertiary amines such as trimethylamine, triethylamine, tripropylamine, tributylamine, triamylamine, triethanolamine, pyridine, N-alkylmorpholine, etc., and tetramethyl, tetraethyl, tetrapropyl, tetrabutyl, tetraamyl, tetrahexyl, benzyltrimethyl, and benzyltriethyl quarternary ammonium hydroxides, etc.

The peptide bond forming reaction can be carried out in an aqueous medium, particularly preferable in a mixed system of water and an organic solvent. In this case, a mixed system of water and an organic

solvent having a high compatibility with water, such as acetone, dioxane, tetrahydrofuran, acetonitrile, and lower alcohols can be advantageously utilized.

A solvent having low compatibility with water, such as ethyl acetate, methylene chloride, chloroform, hexane, ether, etc., generally slows down the reaction rate and is therefore inferior for the yield but even if using such a solvent, the reaction rate can be increased and the yield can be improved by vigorously stirring the mixed system and controlling the pH to a definite value.

The aforesaid reaction can be carried out by adding to an L-proline compound (VI) a base in a equimolar or slightly excess amount thereto, to form previously a salt of the proline, and then adding a solution of the N-carboxy anhydride dissolved in an organic solvent to the solution containing the proline salt thus formed while stirring under cooling. However, the reaction according to this invention is not limited to such a reaction mode but can be performed in various different modes.

It is preferred to use the L-proline in an at least equimolar amount (usually from 1 to 1.5 mol times) to the amount of the N-carboxy anhydride for improving the yield and simplifying the separation procedure of the desired product or isomer.

There is no particular restriction on the reaction temperature and the reaction proceeds smoothly in the range of from -20 °C to room temperature but the reaction at a relatively low temperature is preferred. The reaction time depends upon the reaction temperature but at a temperature about 0 °C, it is sufficient to react of from 10 minutes to 20 minutes.

The reaction can be stopped by adding a mineral acid to the reaction system to acidify the reaction system, whereby carbamic acid which is formed as an intermediate is decomposed (decarboxylation).

The $N^2$-(1-substituted-3-phenylpropyl)-L-lysyl-L-proline derivative (III) formed or, specifically, (XXI), (XXIII) or (XXVI), can be easily isolated by an ordinary extractive separation operation, for example, by concentrating the stopped reaction mixture under reduced pressure to distill off the organic solvent, adjusting the pH of the system to about 4.5, extracting the product with an organic solvent such as methylene chloride, and then concentrating the extract under reduced pressure.

The $N^2$-(1-substituted -3-phenylpropyl)-L-lysyl-L-proline derivative (XXI), (XXIII), or (XXVI) (or generally: (III)) can be easily converted into lysinopril (VIII) by an acid or alkali hydrolysis.

The following examples are intended to illustrate the present invention and not to limit it in any way.

The quantitative analysis in the examples was carried out by a high performance liquid chromatography (HPLC).

Also, the following conditions were used for the analysis:

| | |
|---|---|
| Column: | Finepak® SIL $C_{18-5}$ (made by Nippon Bunkoo K.K.) 4.6 mm ID X 250 mm. |
| Transfer Phase: | 60 mM phosphoric acid buffer (pH 2.5)/acetonitrile = 55/45 to 80/20 (V/V). |
| Flow Rate: | 1.5 ml/min, 1 ml/min. |
| Detection: | 210 nm |

Example 1

Synthesis of $N^2$-(1(S)-cyano-3-phenylpropyl)-$N^6$-trifluoroacetyl-L-lysine:

In 420 ml of methanol was dissolved 6.2 g of sodium cyanide and after further adding dropwise 29.4 g of $N^6$-trifluoroacetyl-L-lysine and further 16.1 g of $\beta$-phenylpropionaldehyde to the solution, the resultant mixture was stirred for 16 hours at room temperature. After the reaction was completed, 10 ml of concentrated hydrochloric acid was slowly added to the reaction mixture and after adding thereto 420 ml of water, the mixture was stirred for 10 minutes at room temperature. The crystals thus deposited were recovered by filtration, washed by 100 ml of water, and dried in vacuo at 80 °C to provide 22.6 g of the crystals of $N^2$-(1-cyano-3-phenylpropyl)-$N^6$-trifluoroacetyl-L-lysine.

The internal standard analysis (internal standard: benzylhydantoin) obtained by using HPLC on the crystals showed that the purity was 92.4% and the isomer ratio of (1S) isomer to (1R) isomer was 91 : 9.

By recrystallising 20 g of the crystals obtained from a mixture of 250 ml of methanol and 250 ml of water, $N^2$-(1(S)-cyano-3-phenylpropyl)-$N^6$-trifluoroacetyl-L-lysine (purity 99%, SS ratio 99.7%) was obtained.

| | |
|---|---|
| $^1$H-NMR ($CDCl_3$): | $\delta$: 1.33-2.3 (m, 8H), 2.67-3.06 (m, 2H), 3.17-3.83 (m, 4H), 5.42-6.4 (m, 1H), 7.08-7.49 (m, 5H), 8.0-8.37 (m, 1H). |
| IR ($cm^{-1}$, KBr, disk): | 3300, 2940, 1700, 1560, 1180, 1160, 700. |

Example 2

Synthesis of $N^2$-(1(S)-cyano-3-phenylpropyl)-$N^6$-trifluoroacetyl-L-lysine-N-carboxy anhydride:

In a 100 ml round bottom flask equipped with a reflux condenser were placed 3.7 g of $N^2$-(1(S)-cyano-3-phenylpropyl)-$N^6$-trifluoroacetyl-L-lysine and 100 ml of a methylene chloride solution (0.6M) of phosgene and the mixture was refluxed for 15 hours. After the reaction was completed, the greater part of methylene chloride (containing phosgene) was distilled off and then methylene chloride was completely removed under reduced pressure, whereby 3.9 g of $N^2$-(1(S)-cyano-3-phenylpropyl)-$N^6$-trifluoroacetyl-L-lysine-N-carboxy anhydride was obtained.

$^1$H-NMR ($CDCl_3$): δ: 1.1-2.17 (m, 6H), 2.17-2.6 (m, 2H), 2.6-3.03 (m, 2H), 3.12-3.53 (m, 2H), 4.14-5.0 (m, 2H), 6.71-7.6 (m, 6H)
IR ($cm^{-1}$, neat): 3350, 2930, 2250, 1860, 1780, 1720, 1560, 760

Example 3

Synthesis of $N^2$-(1(S)-cyano-3-phenylpropyl)-$N^6$-trifluoroacetyl-L-lysyl-L-proline:

To a solution of 1.15 g of L-proline, 317 mg of sodium hydroxide, and 840 mg of sodium carbonate dissolved in 30 ml of water was added a solution of 4 g of $N^2$-(1(S)-cyano-3-phenylpropyl)-$N^6$-trifluoroacetyl-L-lysine-N-carboxy anhydride dissolved in 30 ml of acetone followed by stirring for one hour at 0 °C. After adjusting the pH to about 1 by adding 6N hydrochloric acid, the pH was adjusted to 4.2 by adding 1N sodium hydroxide and after distilling off acetone therefrom, the aqueous layer which remained was extracted with ether. After drying the extracted ether layer by anhydrous sodium sulfate, the solvent was distilled off under reduced pressure to provide 4.3 g of $N^2$-(1(S)-cyano-3-phenylpropyl)-$N^6$-trifluoroacetyl-L-lysyl-L-proline.

$^1$H-NMR ($CDCl_3$): δ: 1.27-2.4 (m, 12H), 2.53-3.0 (m, 2H), 3.08-3.93 (m, 6H), 4.33-4.67 (m, 1H), 5.83-6.34 (m, 2H), 6.98-7.52 (m, 6H)
IR ($cm^{-1}$, neat): 3300, 2950, 1720, 1640, 1455, 1190, 710

Example 4

Synthesis of $N^2$-(1(S)-carbamyl-3-phenylpropyl)-$N^6$-trifluoroacetyl-L-lysine:

After stirring a mixture of 12 g of $N^2$-(1(S)-cyano-3-phenylpropyl)-$N^6$-trifluoroacetyl-L-lysine and 40 ml of concentrated hydrochloric acid for 5 hours at room temperature, 200 ml of ice-water was added thereto and after adjusting the pH to about 4.5 with an aqueous sodium hydroxide solution while stirring, the mixture was further stirred for 30 minutes at 0 °C. The crystals thus deposited were washed with water, recovered by filtration, and dried in vacuo at 45 °C to provide 8.3 g of $N^2$-(1(S)-carbamyl-3-phenylpropyl)-$N^6$-trifluoroacetyl-L-lysine.

$^1$H-NMR ($CDCl_3$, $DMSOd_6$): δ: 1.3-2.13 (m, 3H), 2.53-2.87 (m, 3H), 3.10-3.45 (m, 5H), 4.15 (br s, 2H), 7.0-7.42 (m, 6H)
IR ($cm^{-1}$, KBr, disk): 3400, 3200, 1700, 1680, 1620, 1550, 1190

Example 5

Synthesis of $N^2$-(1(S)-carbamyl-3-phenylpropyl)-$N^6$-trifluoroacetyl-L-lysine-N-carboxy anhydride:

In a 100 ml round bottom flask equipped with a reflux condenser were placed 4 g of $N^2$-(1(S)-carbamyl-3-phenylpropyl)-$N^6$-trifluoroacetyl-L-lysine and 100 ml of a methylene chloride solution (0.6M) of phosgene followed by refluxing for 15 hours. After the reaction was terminated, the greater part of methylene chloride (containing phosgene) was distilled off and further methylene chloride was completely removed under reduced pressure, whereby 8.7 g of $N^2$-(1(S)-carbamyl-3-phenylpropyl)-$N^6$-trifluoroacetyl-L-lysine-N-carboxy anhydride was obtained.

$^1$H-NMR ($CDCl_3$): δ: 1.0-3.62 (m, 16H), 3.8-4.73 (m, 2H), 6.67-7.53 (m, 6H)
IR ($cm^{-1}$, KBr, disk): 3300, 2930, 1845, 1780, 1610, 760, 700

Example 6

Synthesis of $N^2$-(1(S)-carbamyl-3-phenylpropyl)-$N^6$-trifluoroacetyl-L-lysyl-L-proline:

To a solution of 1.18 g of L-proline, 421 mg of sodium hydroxide, and 862 mg of sodium carbonate dissolved in 30 ml of water was added a solution of 3.7 g of $N^2$-(1(S)-carbamyl-3-phenylpropyl)-$N^6$-trifluoroacetyl-L-lysine-N-carboxy anhydride dissolved in 30 ml of acetone followed by stirring for one hour at 0°C. Then, after adjusting the pH of the mixture to about 4.5 by adding 1N sodium hydroxide, acetone was distilled off and the aqueous layer which remained was washed with ether. After saturating the aqueous layer by the addition of sodium sulfate, the product was extracted with methylene chloride. The extract was dried by anhydrous sodium sulfate and then the solvent was distilled off under reduced pressure from the extract to provide 3.9 g of $N^2$-(1(S)-carbamyl-3-phenylpropyl)-$N^6$-trifluoroacetyl-L-lysyl-L-proline.

| | |
|---|---|
| $^1$H-NMR ($CDCl_3$, $CD_3OD$): | δ: 1.27-2.33 (m, 12H), 2.5-2.93 (m, 4H), 3.07-3.67 (m, 6H), 4.33-4.52 (m, 1H), 7.1-7.33 (m, 6H) |
| IR ($cm^{-1}$, KBr, disk): | 3300, 2960, 1650, 1465, 1230, 1200, 1175, 720 |

Example 7

Synthesis of $N^2$-(1(S)-carbamyl-3-phenylpropyl)-$N^6$-trifluoroacetyl-L-lysyl-L-proline:

To one gram of $N^2$-(1(S)-cyano-3-phenylpropyl)-$N^6$-trifluoroacetyl-L-lysine were added 35 ml of 25N sulfuric acid and 5 ml of ethanol at 0°C followed by stirring for 10 hours at 0°C and after adding thereto 100 ml of water while cooling it well, the pH was adjusted to about 4.5 with 6N sodium hydroxide. The reaction product formed was washed with 100 ml of ether, extracted thrice each with 100 ml of methylene chloride, the extract was dried over anhydrous sodium sulfate, and then the solvent was distilled off to provide 720 ml of $N^2$-(1(S)-carbamyl-3-phenylpropyl)-$N^6$-trifluoroacetyl-L-lysyl-L-proline.

Example 8

Synthesis of $N^2$-(1(S)-ethoxycarbonyl-3-phenylpropyl)-$N^6$-trifluoroacetyl-L-lysine:

To 2 g of $N^2$-(1(S)-cyano-3-phenylpropyl)-$N^6$-trifluoroacetyl-L-lysine was added 40 ml of 6N dry HCl/ethanol at 0°C followed by stirring for 20 hours at 0°C and after adding thereto 100 ml of ice water, the resultant mixture was stirred for 30 minutes. After adjusting the pH of the mixture to about 4.5 with an aqueous sodium hydroxide solution, the reaction mixture was extracted with methylene chloride. The extract was dried over anhydrous sodium sulfate and the solvent was distilled off under reduced pressure to provide an oily product. The oily product thus obtained was separated by silica gel column chromatography (eluate: butanol/acetic acid/water = 30/3/1) to provide 162 mg of crystals of $N^2$-(1(S)-ethoxycarbonyl-3-phenyl-propyl)-$N^6$-trifluoroacetyl-L-lysine.

| | |
|---|---|
| $^1$H-NMR ($CDCl_3$): | δ: 1.3 (t, 3H, J = 7Hz), 1.42-2.25 (m, 8H), 2.5-2.85 (m, 2H), 3.0-3.55 (m, 4H), 4.17 (q, 2H, J = 7Hz), 5.4-5.83 (br. s, 2H), 6.9-7.4 (m, 6H) |
| IR ($cm^{-1}$, KBr, disk): | 3320, 1740, 1700, 1615, 1205, 1170, 750, 700 |
| | m.p.: 137.0 to 138.0°C |
| | $[\alpha]_D^{25}$ = 7.0° (c = 2, ethanol) |

Example 9

Synthesis of $N^2$-(1(S)-ethoxycarbonyl-3-phenylpropyl)-$N^6$-trifluoroacetyl-L-lysine-N-carboxy anhydride:

In a 100 ml round bottom flask equipped with a reflux condenser were placed $N^2$-(1(S)-ethoxycarbonyl-3-phenylpropyl)-$N^6$-trifluoroacetyl-L-lysine and 20 ml of an aqueous methylene chloride solution (0.6M) of phosgene and the mixture was refluxed for 15 hours. After the reaction was terminated, the greater part of methylene chloride (containing phosgene) was distilled off and then methylene chloride was completely removed under reduced pressure to provide 0.92 g of $N^2$-(1(S)-ethoxycarbonyl-3-phenylpropyl)-$N^6$-trifluoroacetyl-L-lysine-N-carboxy anhydride.

| | |
|---|---|
| $^1$H-NMR ($CDCl_3$): | δ: 1.3 (t, 3H, J = 7Hz), 1.16-2.13 (m, 8H), 2.24-2.6 (m, 2H), 2.62-2.98 (m, 2H), 3.14-3.57 (m, 2H), 4.0-4.43 (m, 4H), 6.6-7.0 (m, 1H), 7.12-7.56 (m, 5H) |
| IR ($cm^{-1}$, KBr, disk): | 3350, 2870, 1855, 1780, 1740, 1710, 1560, 1190, 950 |

Example 10

Synthesis of $N^2$-(1(S)-ethoxycarbonyl-3-phenylpropyl)-$N^6$-trifluoroacetyl-L-lysyl-L-proline:

To a solution of 393 mg of L-proline, 137 mg of sodium hydroxide, and 287 mg of sodium carbonate dissolved in 10 ml of water was added a solution of 782 mg of $N^2$-(1(S)-ethoxycarbonyl-3-phenylpropyl)-$N^6$-trifluoroacetyl-L-lysine-N-carboxy anhydride dissolved in 10 ml of acetone and the mixture was stirred for one hour at 0°C. After adjusting the pH to about 1 by adding 6N hydrochloric acid, the pH was adjusted to 4.5 by the addition of 1N sodium hydroxide. The acetone was distilled off from the solution, and the aqueous layer thus formed was extracted with methylene chloride. After drying the extract over anhydrous sodium sulfate, the solvent was distilled off under reduced pressure to provide 832 mg of $N^2$-(1(S)-ethoxycarbonyl-3-phenylpropyl)-$N^6$-trifluoroacetyl-L-lysyl-L-proline as an oily product. The oily product thus obtained was dissolved in 5.5 ml of t-butyl methyl ether by heating to 40°C and after cooling the solution to 5°C for 20 hours, the crystals thus deposited were diluted with 2 ml of cyclohexane followed by stirring for one hour. The crystals thus obtained were recovered by filtration, washed by a small amount of cyclohexane, and dried in vacuo at room temperature to provide 647 mg of the crystals of $N^2$-(1(S)-ethoxycarbonyl-3-phenylpropyl)-$N^6$-trifluoroacetyl-L-lysyl-L-proline.

m.p.: 74.5 to 76.5°C

$[\alpha]_D^{25}$ = -15.4° (c = 1.0, methanol/0.1N HCl (1/1))

1H-NMR($CDCl_3$): δ: 1.28(t, 3H, J=7Hz), 1.4-2.83 (m, 12H), 2,57-2,67(m, 2H), 3.1-3.73(m, 6H), 4.13(q, 2H, J=7Hz), 4.35-4.63(m, 1H), 6.37 (br. s, 1H), 7.0-7.68(m, 6H).

Example 11

Synthesis of $N^2$-(1(S)-carboxy-3-phenylpropyl)-L-lysyl-L-proline:

To 10 ml of 6N sulfuric acid was added 1 g of $N^2$-(1(S)-carbamyl-3-phenylpropyl)-$N^6$-trifluoroacetyl-L-lysine followed by stirring for 15 hours at 80°C, after cooling the mxiture to 0°C, 50 ml of water was added thereto, and after adjusting the pH thereof to 5.5 with an aqueous solution of 2N sodium hydroxide and then adjusting the pH to 7.5 with 1N ammonium hydroxide, the volatile matter was distilled off. To the residue formed was added 10 ml of ethanol, insoluble inorganic salts were filtered off, and after concentrating the filtrate under reduced pressure, the residual solids were purified by LH-20 chromatography (methanol) to provide 632 mg of $N^2$-(1(S)-carboxy-3-phenylpropyl)-L-lysyl-L-proline as solids. The properties of the product coincided with those described in Journal of Pharmaceutical Science, 74, 352(1985).

Example 12

Synthesis of $N^2$-(1(S)-carboxy-3-phenylpropyl)-L-lysyl-L-proline:

To a mixture of 5 ml of ethanol, 10 ml of water, and 25 ml of concentrated sulfuric acid was added 1 g of $N^2$-(1(S)-cyano-3-phenylpropyl)-$N^6$-trifluoroacetyl-L-lysine followed by stirring for 45 minutes at room temperature.

To the reaction mixture was added 115 ml of water and the mixture was refluxed for 24 hours at 80°C. When the reaction was over, the pH was adjusted to 7.5 with 5N ammonium hydroxide, the volatile matters were distilled off, and the residue formed was purified as in Example 11 to provide 320 mg of $N^2$-(1(S)-carboxy-3-phenylpropyl)-L-lysyl-L-proline.

**Claims**

**1.** An $N^2$-(1-substituted-3-phenylpropyl)-L-lysine derivative represented by formula (I)

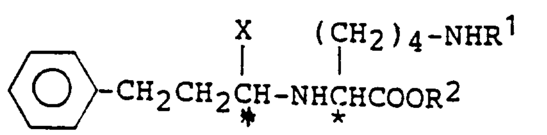

(I)

wherein $R^1$ represents an acyl type or urethane type protective group; $R^2$ represents a hydrogen atom, an alkyl group, or an aralkyl group; X represents a cyano (CN) group or an aminocarbonyl ($CONH_2$) group; and the mark * represents the (S) configuration of the asymmetric carbon atom.

**2.** An $N^2$-(1-substituted-3-phenylpropyl)-L-lysine-N-carboxy anhydride represented by formula (II)

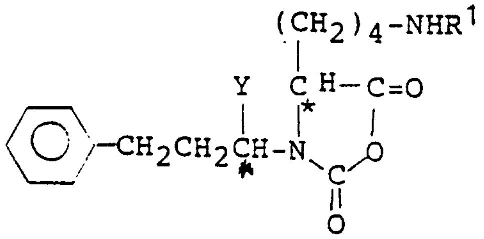

(II)

wherein $R^1$ represents an acyl type or urethane type protective group; Y represents a cyano (CN) group, an aminocarbonyl ($CONH_2$) group, or an alkoxycarbonyl group shown by the formula COOW (wherein W represents an alkyl group having from 1 to 4 carbon atoms); and the mark * represents the (S) configuration of the asymmetric carbon atom.

**3.** The derivative as claimed in claim 1 or 2, wherein $R^1$ is a trifluoroacetyl group.

**4.** A process of producing an $N^2$-(1-cyano-3-phenylpropyl)-L-lysine derivative represented by formula (V)

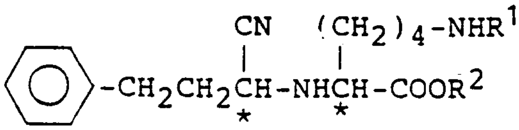

(V)

wherein $R^1$ represents an acyl type or urethane type protective group; $R^2$ represents a hydrogen atom, an alkyl group, or an aralkyl group, and the mark * represents the (S)configuration of the asymmetric carbon atom, which comprises reacting β-phenylpropionaldehyde, a cyano compound, and a compound represented by formula (IV)

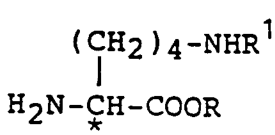

(IV)

wherein $R^1$, $R^2$, and the mark * have the same significance as defined above in a protonic solvent at a temperature of from -40 to 80 °C.

**5.** A process of producing an $N^2$-(1-substituted-3-phenylpropyl)-L-lysyl-L-proline derivative represented by formula (VII)

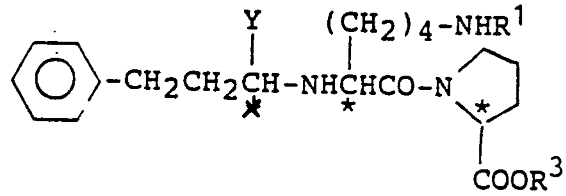

(VII)

wherein $R^1$ represents an acyl type or urethane type protective group; $R^3$ represents a hydrogen atom, an alkyl group, or an aralkyl group; Y represents a cyano group, an aminocarbonyl group, or an alkoxycarbonyl group shown by the formula COOW (wherein W represents an alkyl group having from 1 to 4 carbon atoms); and the mark * represents the (S) configuration of the asymmetric carbon atoms, which comprises reacting $N^2$-(1-substituted 3-phenylpropyl)-L-lysine-N-carboxy anhydride represented by formula (II)

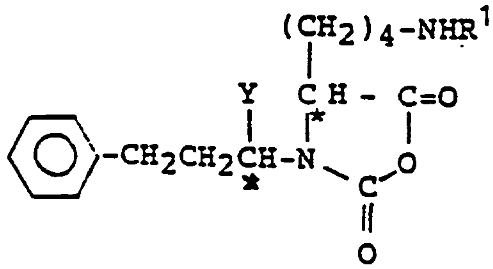

(II)

wherein $R^1$, Y, and the mark * have the same significance as defined above and a L-proline represented by formula (VI) or a derivative thereof

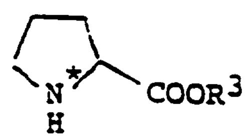

(VI)

wherein $R^3$ and the mark * have the same significance as defined above.

6. A process according to claim 5, which further comprises hydrolyzing the $N^2$-(1-subtituted-3-phenylpropyl)-L-lysyl-L-proline derivative represented by formula (VII) to obtain an $N^2$-(1-(S)-carboxy-3-phenylpropyl)-L-lysyl-proline (lysinopril) represented by formula (VIII)

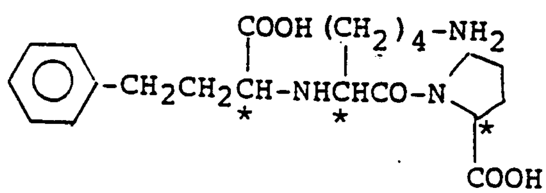

(VIII)

wherein the mark * represents the (S) configuration of the asymmetric carbon atom.

7. Use of an $N^2$-(1-(S)-substituted-3-phenylpropyl)-L-lysine-N-carboxyanhydride represented by formula (II)

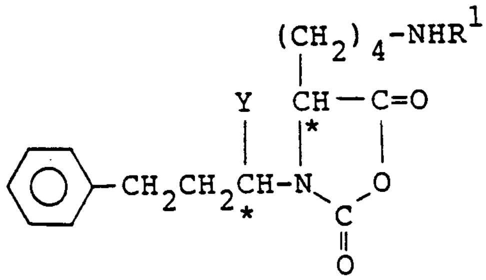

(II)

wherein $R^1$ represents an acyl type or urethane type protective group; Y represents a cyano (CN) group, an aminocarbonyl ($CONH_2$) group, or an alkoxycarbonyl group shown by the formula COOW (wherein W represents an alkyl group having from 1 to 4 carbon atoms); and the mark * represents the (S) configuration of the asymmetric carbon atom, for producing $N^2$-(1-(S)-carboxy-3-phenylpropyl)-L-lysyl-proline (lysinopril) represented by formula (VIII)

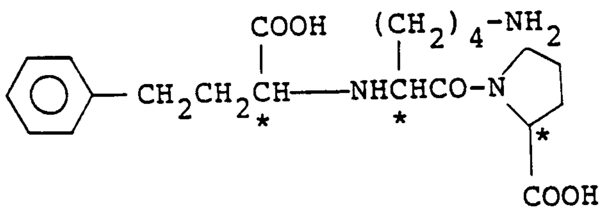

(VIII)

wherein the mark * represents the same as the above.

## Patentansprüche

**1.** $N^2$-(1-substituiertes 3-Phenylpropyl)-L-lysinderivat der Formel (I)

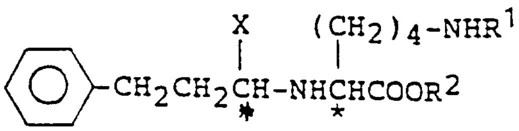

(I)

in der $R^1$ eine Schutzgruppe vom Acyl- oder Urethantyp bedeutet; $R^2$ ein Wasserstoffatom, einen Alkylrest oder einen Aralkylrest bedeutet; X eine Cyanogruppe (CN) oder eine Aminocarbonylgruppe ($CONH_2$) bedeutet; und das Zeichen * die (S)-Konfiguration des asymmetrischen Kohlenstoffatoms bedeutet.

**2.** $N^2$-(1-substituiertes 3-Phenylpropyl)-L-lysin-N-carbonsäureanhydrid der Formel (II)

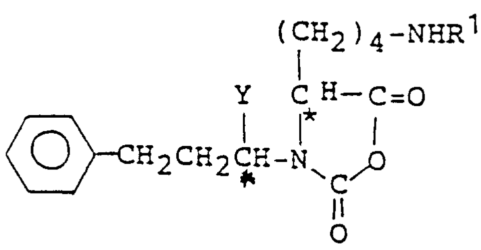

(II)

in der $R^1$ eine Schutzgruppe vom Acyl- oder Urethantyp bedeutet; Y eine Cyanogruppe (CN), eine

Aminocarbonylgruppe ($CONH_2$) oder einen Alkoxycarbonylrest der Formel COOW (in der W einen Alkylrest mit 1 bis 4 Kohlenstoffatomen bedeutet) bedeutet; und das Zeichen * die (S)-Konfiguration des asymmetrischen Kohlenstoffatoms bedeutet.

**3.** Derivat nach Anspruch 1 oder 2, wobei $R^1$ eine Trifluoracetylgruppe bedeutet.

**4.** Verfahren zur Herstellung eines $N^2$-(1-Cyano-3-phenylpropyl)-L-lysinderivates der Formel (V)

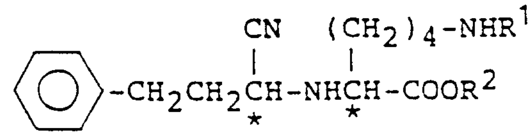

(V)

in der $R^1$ eine Schutzgruppe vom Acyl- oder Urethantyp bedeutet; $R^2$ ein Wasserstoffatom, einen Alkylrest oder einen Aralkylrest bedeutet; und das Zeichen * die (S)-Konfiguration des asymmetrischen Kohlenstoffatoms bedeutet, umfassend die Umsetzung von $\beta$-Phenylpropionaldehyd, einer Cyanoverbindung und einer Verbindung der Formel (IV)

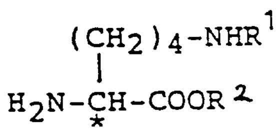

(IV)

in der $R^1$, $R^2$ und das Zeichen * die vorstehend angegebene Bedeutung haben, in einem protischen Lösungsmittel bei einer Temperatur von -40 bis 80 °C.

**5.** Verfahren zur Herstellung eines $N^2$-(1-substituierten 3-Phenylpropyl)-L-lysyl-L-prolinderivates der Formel (VII)

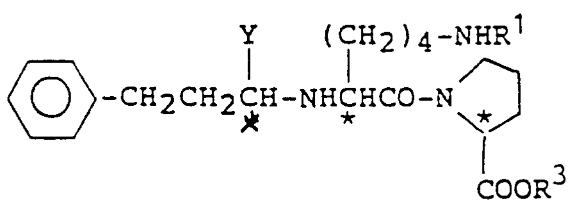

(VII)

in der $R^1$ eine Schutzgruppe vom Acyl- oder Urethantyp bedeutet; $R^3$ ein Wasserstoffatom, einen Alkylrest oder einen Aralkylrest bedeutet; Y eine Cyanogruppe, eine Aminocarbonylgruppe oder einen Alkoxycarbonylrest der Formel COOW (in der W einen Alkylrest mit 1 bis 4 Kohlenstoffatomen bedeutet) bedeutet; und das Zeichen * die (S)-Konfiguration des asymmetrischen Kohlenstoffatoms bedeutet, umfassend die Umsetzung eines $N^2$-(1-substituierten 3-Phenylpropyl)-L-lysin-N-carbonsäureanhydrids der Formel (II)

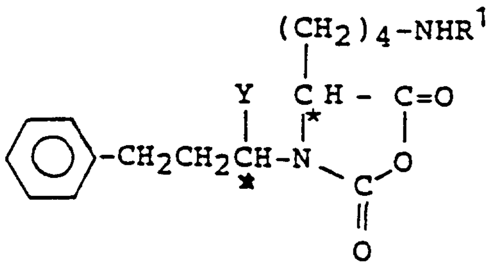

(II)

in der $R^1$, Y und das Zeichen * die vorstehend angegebene Bedeutung haben, mit einem L-Prolin der Formel (VI) oder einem Derivat davon

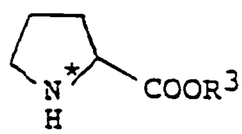

(VI)

wobei $R^3$ und das Zeichen * die vorstehend angegebene Bedeutung haben.

**6.** Verfahren nach Anspruch 5, das ferner die Hydrolyse des $N^2$-(1-substituierten 3-Phenylpropyl)-L-lysyl-L-prolinderivates der Formel (VII) zu einem $N^2$-((S)-1-Carboxy-3-phenylpropyl)-L-lysyl-prolin (Lysinopril) der Formel (VIII)

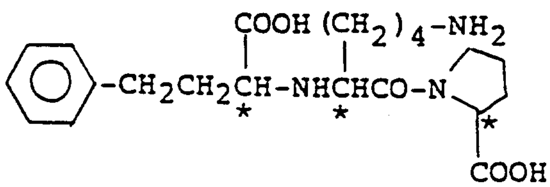

(VIII)

in der das Zeichen * die (S)-Konfiguration des asymmetrischen Kohlenstoffatoms bedeutet, umfaßt.

**7.** Verwendung eines $N^2$-((S)-1-substituierten 3-Phenylpropyl)-L-lysin-N-carbonsäureanhydrids der Formel (II)

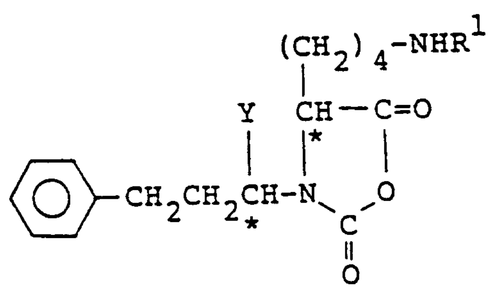

(II)

in der $R^1$ eine Schutzgruppe vom Acyl- oder Urethantyp bedeutet; Y eine Cyanogruppe (CN), eine Aminocarbonylgruppe ($CONH_2$) oder einen Alkoxycarbonylrest der Formel COOW (in der W einen Alkylrest mit 1 bis 4 Kohlenstoffatomen bedeutet) bedeutet; und das Zeichen * die (S)-Konfiguration des asymmetrischen Kohlenstoffatoms bedeutet, zur Herstellung von $N^2$-((S)-1-Carboxy-3-phenylpropyl)-L-lysyl-prolin (Lysinopril) der Formel (VIII)

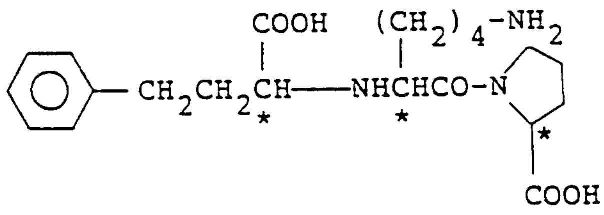

(VIII)

in der das Zeichen * die vorstehend angegebene Bedeutung hat.

**Revendications**

1. Dérivé de $N^2$-(1-substitué-3-phénylpropyl)-L-lysine représenté par la formule (I)

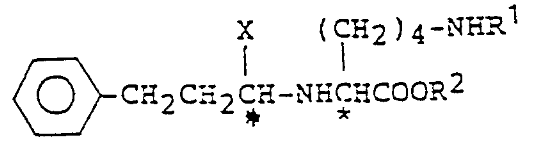

(I)

dans laquelle $R^1$ représente un groupe protecteur du type acyle ou du type uréthane; $R^2$ représente un atome d'hydrogène, un radical alkyle ou un radical aralkyle; X représente un radical cyano (CN) ou un radical aminocarbonyle ($CONH^2$); et le signe * représente la configuration (S) de l'atome de carbone asymétrique.

2. Anhydride de $N^2$-(1-substitué-3-phénylpropyl)-L-lysine-N-carboxy représenté par la formule (II)

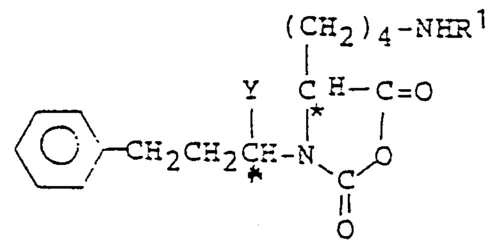

(II)

dans laquelle $R^1$ représente un radical protecteur du type acyle ou du type uréthane; Y représente un radical cyano (CN), un radical aminocarbonyle ($CONH^2$) ou un radical alkoxycarbonyle représenté par la formule COOW (où W représente un radical alkyle possédant de 1 à 4 atomes de carbone); et le signe * représente la configuration (S) de l'atome de carbone asymétrique.

3. Dérivé selon la revendication 1 ou 2, dans lequel $R^1$ est un radical trifluoroacétyle.

4. Procédé de production d'un dérivé de $N^2$-(1-cyano-3-phénylpropyl)-L-lysine représenté par la formule (V),

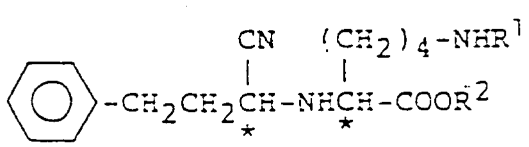

(V)

dans laquelle $R^1$ représente un groupe protecteur du type acyle ou du type uréthane; $R^2$ représente un atome d'hydrogène, un radical alkyle ou un radical aralkyle, et le signe * représente la configuration (S) de l'atome de carbone asymétrique, qui consiste à faire réagir du $\beta$-phénylpropionaldéhyde, un composé cyano et un composé représenté par la formule (IV)

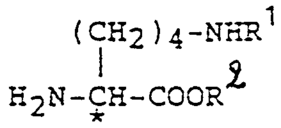

(IV)

dans laquelle $R^1$ et $R^2$, et le signe * ont la même signification que celle définie plus haut, dans un solvant protonique à une température de -40 à 80 ° C.

**5.** Procédé de production d'un dérivé de $N^2$-(1-substitué-3-phénylpropyl)-L-lysyl-L-proline représenté par la formule (VII)

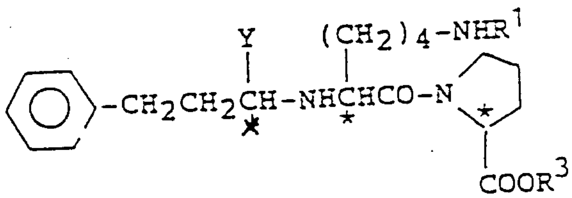

(VII)

dans laquelle $R^1$ représente un radical protecteur du type acyle ou du type uréthane; $R^3$ représente un atome d'hydrogène, un radical alkyle ou un radical aralkyle; Y représente un radical cyano, un radical aminocarbonyle ou un radical alkoxycarbonyle représenté par la formule COOW (où W représente un radical alkyle possédant de 1 à 4 atomes de carbone); et le signe * représente la configuration (S) de l'atome de carbone asymétrique, qui consiste à faire réagir un anhydride de $N^2$-(1-substitué-3-phénylpropyl)-L-lysine-N-carboxy représenté par la formule (II)

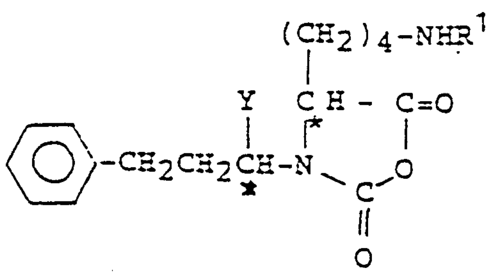

(II)

dans laquelle $R^1$, Y et le signe * ont la même signification que celle définie plus haut,
et une L-proline représentée par la formule (VI), ou un dérivé de celle-ci

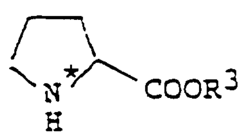

(VI)

dans laquelle $R^3$ et le signe * ont la même signification que celle définie plus haut.

**6.** Procédé selon la revendication 5, qui consiste de plus à hydrolyser le dérivé de $N^2$-(1-substitué-3-phénylpropyl)-L-lysyl-L-proline représenté par la formule (VII) pour obtenir une $N^2$-(1(S)-carboxy-3-phénylpropyl)-L-lysyl-proline (lysinopril) représentée par la formule (VIII)

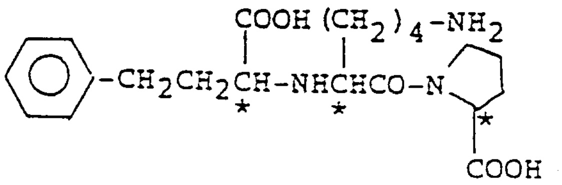

(VIII)

dans laquelle le signe * représente la configuration (S) de l'atome de carbone asymétrique.

**7.** Utilisation d'un anhydride de $N^2$-(1-(S)-substitué-3-phénylpropyl)-L-lysine-N-carboxy représenté par la formule (II)

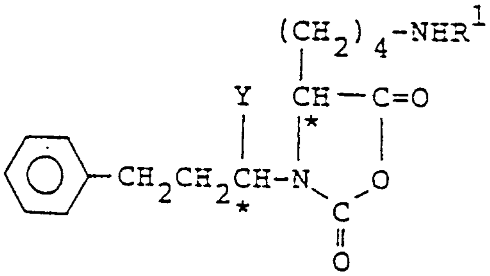

(II)

dans laquelle $R^1$ représente un radical protecteur du type acyle ou du type uréthane; Y représente un radical cyano (CN), un radical aminocarbonyle ($CONH^2$) ou un radical alkoxycarbonyle représenté par la formule COOW (où W représente un radical alkyle possédant de 1 à 4 atomes de carbone); et le signe * représente la configuration (S) de l'atome de carbone asymétrique, pour produire la $N^2$-(1-(S)-carboxy-3-phénylpropyl)-L-lysyl-proline (lysinopril) représentée par la formule (VIII)

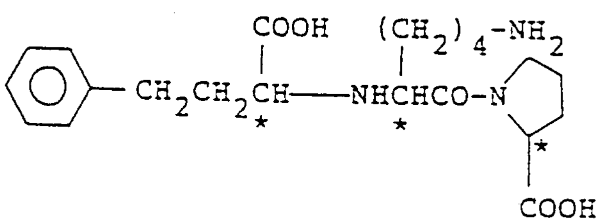

(VIII)

dans laquelle le signe * représente la même chose que ci-dessus.